# EUROPÄISCHE PATENTANMELDUNG

(11) **EP 4 230 250 A1**
(43) Veröffentlichungstag der Anmeldung: **23.08.2023**
(21) Anmeldenummer: 23173294.2
(22) Anmeldetag: 13.04.2021
(51) Int. Cl.: A61M 37/00, A61C 17/34

(54) **MICRONEEDLING-HANDGERÄT ZUM LOKALEN AUFSTECHEN EINER HAUT UND ARTIKEL**

(62) Teilanmeldung aus: 21168112.7
(71) Anmelder: MT.DERM GmbH, 12307 Berlin (DE)
(72) Erfinder: Scherkowski, Dirk, 12489 Berlin (DE)
(74) Vertreter: Bittner, Thomas L.

(57) **Zusammenfassung**

Die Erfindung betrifft ein Microneedling-Handgerät (1) zum lokalen Aufstechen einer Haut mit: einem Gehäuse (2); einer Antriebseinrichtung, die in dem Gehäuse (2) angeordnet und eingerichtet ist, eine Antriebskraft bereitzustellen; einer Hautstecheinrichtung (6); einem Microneedling-Stechwerkzeug (8), das an der Hautstecheinrichtung (6) gebildet und mit der Antriebseinrichtung verbunden ist, derart, dass das Microneedling-Stechwerkzeug (8) mittels der Antriebskraft im Betrieb mit einer Wiederholfrequenz vor und zurück bewegbar ist; und einer Nadelplatte (9), die an dem Microneedling-Stechwerkzeug (8) gebildet ist und bei der über eine vorderseitige Anwendungsfläche (11) verteilt mehrere Stechnadeln (10) angeordnet sind. Das Microneedling-Stechwerkzeug (8) ist mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar. Weiterhin sind eine Hautstecheinrichtung (6) für ein Microneedling-Handgerät sowie ein Artikel geschaffen wobei die Hautstecheinrichtung (6) an dem Gehäuse (2) lösbar angeordnet ist und wobei die Antriebseinrichtung mit einer Antriebseinrichtung für eine elektrische Zahnbürste gebildet ist, welche eingerichtet ist, hieran wahlweise die Hautstecheinrichtung (6) und einen mittels der Antriebseinrichtung betreibbaren Zahnbürstenaufsatz lösbar zu koppeln. (Fig. 1)

## Beschreibung

Die Erfindung betrifft ein Microneedling-Handgerät zum lokalen Aufstechen einer Haut und einen Artikel.

### Hintergrund

Beim Microneedling wird die Haut mit mehreren Stechnadeln in einem flächigen Hautabschnitt gleichzeitig lokal aufgestochen. Die mehreren Stechnadeln sind an einem Microneedling-Stechwerkzeug eines Handgeräts über eine Anwendungsfläche verteilt angeordnet, zum Beispiel an einer Nadelplatte.

Das Microneedling verursacht aufgrund des lokalen Aufstechens feinste Verletzungen der Haut, die die Haut als Wunden registriert. In der Haut werden dann verschiedene Botenstoffe und Wachstumsfaktoren ausgeschüttet, um die Wundheilung anzuregen. Dieser Vorgang regt auch die Produktion von Kollagen, Elastin und Hyaluronsäure an, die Straffung und Elastizität der Haut fördern. Microneedling kann mit und ohne Substanz- oder Wirkstoffeintrag in die Haut ausgeführt werden. Die Haut wird lokal aufgestochen, um positive Heilungsreaktionen auszulösen, was wahlweise mittels Substanzeintrag unterstützt werden kann.

Zum Anwenden des Microneedling sind beispielsweise manuelle Hautstecheinrichtungen in stempelartiger Ausführung bekannt, bei der das Microneedling-Stechwerkzeug mit einem Stempel gebildet ist, bei dem über eine Anwendungsfläche verteilt mehrere Stechnadeln angeordnet sind. Bei anderen manuellen Handgeräten sind die Stechnadeln über die Mantelfläche einer Walze verteilt, die zum lokalen Aufstechen der Haut über diese gerollt wird.

US 2017/0358810 A1 betrifft ein dermatologisches Microneedling-Gerät und US 2019/0351205 A1 ein dermatologisches Behandlungssystem.

US 2014/0018835 A1 beschreibt ein Handgerät zu Stechen einer menschlichen oder Tierischen Haut sowie ein Nadelmodul für ein solches Handgerät.

DE 10 2016 003 599 A1 betrifft einen elektrischen linearen repetitiven Impulsantrieb zum Betrieb von Geräten.

US 2021/0005372 A1 betrifft ein Kleingerät zur Körperpflege.

Weiterhin sind Microneedling-Handgeräte in Form eines sogenannten Pens bekannt, bei dem ein Microneedling-Stechwerkzeug einer Hautstecheinrichtung mit einer Nadelplatte gebildet ist, an der die mehreren Stechnadeln im Bereich einer Anwendungsfläche angeordnet sind. Das Microneedling-Stechwerkzeug wird mit Hilfe eines elektrischen Motors einer Antriebseinrichtung automatisiert vor- und zurückbewegt. Mit Hilfe des elektrischen Motors der Antriebseinrichtung wird eine Antriebskraft bereitgestellt, die auf das Microneedling-Stechwerkzeug eingekoppelt wird, um die Nadelplatte mit der Anwendungsfläche und den hierauf flächig verteilten Stechnadeln vor und zurück zu bewegen, derart, dass Nadelspitzen der Stechnadeln in Bezug auf eine vorderseitige Gehäuseöffnung eines Gehäuses des Handgeräts zwischen einer ausgefahrenen und einer eingefahrenen Stellung vor- und zurückbewegt werden. Eine Nadelplatte, an welcher die Stechnadeln angeordnet sind, ist von einem vorderen Gehäuseabschnitt umgeben. Ein vorderseitiger Rand des Gehäuseabschnitts, welcher die Gehäuseöffnung umgibt, durch welche hindurch die Nadelspitzen der Stechnadeln aus- und eingefahren werden, wird im Betrieb auf die Hautoberfläche aufgelegt, welche hierdurch in dem Anwendungsbereich fixiert wird, um dann mittels Vor- und Zurückbewegen der Nadelspitzen die (gespannte) Hautoberfläche lokal aufzustechen. Der die vorderseitige Gehäuseöffnung umgebende Rand dient hier dem Fixieren und Festhalten des Hautabschnitts, in welchen dann lokal mittels der Stechnadeln an der Nadelplatte eingestochen wird.

### Zusammenfassung

Aufgabe der Erfindung ist es, ein Microneedling-Handgerät zum lokalen Aufstechen einer Haut anzugeben, welches eine verbesserte Anwendung des Microneedling ermöglicht.

Gelöst wird die Aufgabe durch ein Microneedling-Handgerät zum lokalen Aufstechen einer Haut nach Anspruch 1. Weiterhin sind Hautstecheinrichtung ein Microneedling-Handgerät sowie ein Artikel nach den nebengeordneten Ansprüchen 16 und 17 geschaffen. Ausgestaltungen sind Gegenstand von abhängigen Unteransprüchen.

Nach einem Aspekt ist ein Microneedling-Handgerät zum lokalen Aufstechen einer Haut geschaffen, welches Folgendes aufweist: ein Gehäuse; eine Antriebseinrichtung, die in dem Gehäuse angeordnet und eingerichtet ist, eine Antriebskraft bereitzustellen; eine Hautstecheinrichtung, die in dem Gehäuse angeordnet ist; ein Microneedling-Stechwerkzeug, das an der Hautstecheinrichtung gebildet und mit der Antriebseinrichtung verbunden ist, derart, dass das Microneedling-Stechwerkzeug mittels der Antriebskraft im Betrieb mit einer Wiederholfrequenz vor und zurück bewegbar ist; und eine Nadelplatte, die an dem Microneedling-Stechwerkzeug gebildet ist und bei der über eine vorderseitige Anwendungsfläche verteilt mehrere Stechnadeln angeordnet sind. Das Microneedling-Stechwerkzeug ist mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar.

Nach einem weiteren Aspekt ist eine Hautstecheinrichtung mit einem Gehäuse geschaffen, die zum Ausbilden eines Microneedling-Handgeräts an eine Antriebseinrichtung koppelbar ist und Folgendes aufweist: ein Microneedling-Stechwerkzeug, das an der Hautstecheinrichtung gebildet und mit der Antriebseinrichtung verbindbar ist, derart, dass das Microneedling-Stechwerkzeug mittels einer von der Antriebseinrichtung bereitgestellten Antriebskraft im Betrieb mit einer Wiederholfrequenz vor und zurück bewegbar ist; und eine Nadelplatte, die an dem Microneedling-Stechwerkzeug gebildet ist und bei der über eine vorderseitige Anwendungsfläche verteilt mehrere Stechnadeln angeordnet sind. Das Microneedling-Stechwerkzeug ist zwischen einer vorderen Arbeitsstellung und einer im Vergleich hierzu zurückgestellten hinteren Arbeitsstellung vor und zurück bewegbar ist, wobei zumindest die mehreren Stechnadeln der Nadelplatte hierbei in der vorderen Arbeitsstellung und der hinteren Arbeitsstellung des Microneedling-Stechwerkzeugs vollständig außerhalb des Gehäuses und so freiliegend angeordnet sind.

Nach einem weiteren Aspekt ist ein Artikel geschaffen, der Folgendes aufweist: eine Antriebseinrichtung, die in einem Gehäuse angeordnet ist, welches wahlweise ein Handstück ausbildend ausgeführt ist; einen Zahnbürstenaufsatz, welcher zum Ausbilden einer elektrischen Zahnbürste mit dem Gehäuse lösbar und so an die Antriebseinrichtung koppelnd verbunden werden kann; und eine Hautstecheinrichtung, welche zum Ausbilden eines Microneedling-Handgeräts mit dem Gehäuse lösbar und so an die Antriebseinrichtung koppelnd verbunden werden kann.

Beim Bewegen zwischen der vorderen Arbeitsstellung und der hinteren Arbeitsstellung können in einer Ausführung zumindest die mehreren Stechnadeln der Nadelplatte in der vorderen Arbeitsstellung und der hinteren Arbeitsstellung des Microneedling-Stechwerkzeugs vollständig außerhalb des Gehäuses und so freiliegend angeordnet sein. Das Microneedling-Handgerät ist so konstruktiv eingerichtet, ein weiter verbessertes Microneedling zu ermöglichen. Da zumindest die mehreren Stechnadeln der Nadelplatte an dem Microneedling-Stechwerkzeug sowohl in der vorderen Arbeitsstellung wie auch in der hinteren Arbeitsstellung jeweils vollständig außerhalb des wahlweise ein- oder mehrstückigen Gehäuses, insbesondere außerhalb eines Gehäusebauteils der Hautstecheinrichtung, und so freiliegend angeordnet sind, kann der lokal aufzustechende Hautabschnitt aufgrund der repetierenden Bewegung der Nadelplatte mit den mehreren Stechnadeln selbst in Schwingungen versetzt werden, wird also nicht vom Gehäuse des Microneedling-Handgeräts hieran gehindert, was ein effizientes und möglichst schmerzfreies Einstechen der mehreren Stechnadeln in die Haut ermöglicht.

Im Betrieb kann weiterhin zumindest die vorderseitige Anwendungsfläche sowohl in der vorderen Arbeitsstellung wie auch in der zurückgestellten hinteren Arbeitsstellung des Microneedling-Stechwerkzeugs vollständig außerhalb des Gehäuses und so freiliegend angeordnet sein, insbesondere außerhalb eines von Gehäuseabschnitten des Gehäuses zumindest teilweise umgebenen Gehäuseinnenraum. Auf diese Weise ist das Microneedling-Handgerät ergänzend konstruktiv eingerichtet, im Betrieb beim Microneedling das freie Schwingen des bearbeiteten Hautabschnitts zuzulassen.

Bei dem Microneedling-Stechwerkzeug kann in alternativen Ausgestaltungen die Nadelplatte frei von einem die Nadelplatte zumindest teilweise umgreifenden Gehäuseabschnitt des Gehäuses gebildet sein.

Die Nadelplatte kann an einem Gehäuseabschnitt des Gehäuses angeordnet sein, welcher im Betrieb beim Vor- und Zurückbewegen des Microneedling-Stechwerkzeugs mit diesem zusammen mitbewegt wird. Hierbei kann es sich um ein vorderes Gehäuseteil des Gehäuses handeln. Die Nadelplatte kann hierbei auf einer Außenseite des Gehäuseabschnitts gebildet sein, mit der die Anwendungsfläche bereitgestellt ist. Die Nadelplatte kann an einem vorderen Gehäuseabschnitt des Gehäuses angeformt sein, welcher im Betrieb zusammen mit dem Microneedling-Stechwerkzeug vor und zurück bewegt wird. Beispielsweise kann die Nadelplatte als eine Frontplatte des Gehäuseabschnitts angeformt sein. Die Nadelplatte kann lösbar oder nicht-lösbar an dem Gehäuseabschnitt angeordnet sein.

Die Nadelplatte kann an dem Gehäuseabschnitt des Gehäuses einstückig angeformt sein. Hierbei kann ein seitlicher Wandabschnitt des Gehäuseabschnitts einstückig mit einer die Anwendungsfläche bereitstellenden Frontplatte einstückig ausgeführt sein. Der Gehäuseabschnitt kann so das Microneedling-Stechwerkzeug bildend ausgeführt sein, insbesondere die Nadelplatte.

Bei verschiedenen Ausführungsformen können die mehreren Stechnadeln im Bereich der Anwendungsfläche auf die Nadelplatte aufgebracht sein. Alternativ kann vorgesehen sein, dass die Stechnadeln in Öffnungen der Nadelplatte angeordnet sind, beispielsweise eingelötet oder eingeklebt sind, wobei sich die Stechnadeln zumindest abschnittsweise durch die Öffnungen hindurch erstrecken können. Es kann vorgesehen sein, dass die Stechnadeln nach dem Durchgang durch die Öffnungen an einem in Bezug auf die Anwendungsfläche der Nadelplatte rückseitigen Nadelhalterelement aufgenommen sind, welches als Teil des Microneedling-Stechwerkzeugs oder der Nadelplatte ausbildbar ist.

Alternativ können die Stechnadeln einstückig an der Nadelplatte angeformt sein. So können die Stechnadeln zum Beispiel mittels gestanzter und gebogener Blechelemente gebildet sein.

Stechnadeln im Sinne der vorliegenden Offenbarung sind allgemein scharfe bzw. spitze, von der Nadelplatte hervorstehende Volumenelemente, die hinsichtlich Form und Festigkeit für eine wiederholte Penetration der Haut eingerichtet sind. Die Stechnadeln können aus einem Vollmaterial sein, im Unterschied zur Kanüle (Hohlnadel).

Ein vorderes Gehäuseteil, welches der Hautstecheinrichtung zugeordnet ist, kann rückwärtig hülsenförmig gestaltet sein und einen hinteren Gehäuseteil zumindest teilweise umschließen oder umgreifen, welcher der Antriebseinrichtung zugeordnet ist. Bei dieser oder anderen Ausführungsformen kann mit dem vorderen Gehäuseteil, welches vom hinteren Gehäuseteil abnehmbar sein kann, ein Gehäusebauteil der Hautstecheinrichtung gebildet sein, zum Beispiel ein Modulgehäuse eines Stechmoduls.

Die Hautstecheinrichtung ist an dem Gehäuse lösbar angeordnet. Auf diese Weise kann das Microneedling-Stechwerkzeug mit einem zugeordneten Gehäuseteil wechselbar an dem Gehäuse angeordnet werden.

Die Antriebseinrichtung ist mit einer Antriebseinrichtung für eine elektrische Zahnbürste gebildet, welche eingerichtet ist, hieran wahlweise die Hautstecheinrichtung und einen mittels der Antriebseinrichtung betreibbaren Zahnbürstenaufsatz lösbar zu koppeln. Antriebseinrichtungen für elektrische Zahnbürsten sind als solche in verschiedenen Ausführungen bekannt. Das Gehäuse der Antriebseinrichtung bildet häufig ein Handstück der elektrischen Zahnbürste, welches der Nutzer beim Benutzen umgreift. Die Antriebseinrichtung stellt eine Antriebskraft / -bewegung für den elektrischen Betrieb des Zahnbürstenaufsatzes bereit. Der Zahnbürstenaufsatz ist abnehmbar. Die Antriebseinrichtung der elektrischen Zahnbürste kann dann zum Ausbilden des Microneedling-Handgeräts verwendet werden, indem die Hautstecheinrichtung auf die Antriebseinrichtung der elektrischen Zahnbürste lösbar aufgesetzt wird, so dass die bereitgestellte Antriebskraft auf das Microneedling-Stechwerkzeug eingeleitet werden kann, derart, dass das Microneedling-Stechwerkzeug mittels der Antriebskraft im Betrieb mit einer Wiederholfrequenz vor und zurück bewegt wird.

Das Microneedling-Stechwerkzeug kann zusammen mit dem Gehäuseabschnitt von dem Gehäuse lösbar oder abnehmbar sein. Das Microneedling-Stechwerkzeug und / oder die Hautstecheinrichtung mit dem Microneedling-Stechwerkzeug, sei es separat oder gemeinsam mit dem Gehäuseabschnitt, kann mittels einer Steckverbindung oder einer Schraubverbindung lösbar montiert sein. So kann das Microneedling-Stechwerkzeug gemeinsam mit dem Gehäuseabschnitt auf einen zugeordneten Gehäuseabschnitt aufsteckbar sein, insbesondere einen Gehäuseabschnitt, der die Antriebseinrichtung aufnimmt.

Das Microneedling-Stechwerkzeug kann durch eine Öffnung an einem vorderseitigen Gehäuseende des Gehäuses hindurch in eine im Vergleich zur zurückgestellten hinteren Arbeitsstellung weiter zurückgestellte Nichtarbeitsstellung verlagerbar sein, in welcher zumindest die vorderseitige Anwendungsfläche der Nadelplatte gegenüber der Öffnung zurücksteht. Bei dieser Ausführungsform kann das Microneedling-Stechwerkzeug im Nicht-Betrieb in die zurückgestellte Nichtarbeitsstellung verlagert werden, beispielsweise derart, dass die mehreren Stechnadeln hinter einer Öffnungsfläche der (Gehäuse-)Öffnung am vorderseitigen Gehäuseende angeordnet und somit mittels des Gehäuses geschützt sind.

Alternativ oder ergänzend kann bei dieser oder anderen Ausführungsformen vorgesehen sein, auf das vorderseitige Gehäuseende eine Schutzkappe lösbar aufzusetzen. Das Gehäuse kann die Öffnung teilweise oder vollständig umgreifen. In der zurückgestellten Nichtarbeitsstellung kann die Nadelplatte umlaufend teilweise oder vollständig von dem Gehäuse umgeben sein, so dass die Nadelplatte dann im Gehäuseinnenraum angeordnet ist.

Die Nadelplatte kann beim Verlagern durch die (Gehäuse-)Öffnung hindurch ein- oder mehrseitig von einem die Öffnung umgebenden Rand beabstandet sein. Bei dieser Ausführungsform wird die Nadelplatte in dem ein- oder mehrseitig ausgebildeten Abstandsbereichen berührungskontaktfrei durch die Öffnung hindurch verlagert. Insbesondere findet so in den Abstandsbereichen keine Führung der Nadelplatte am Gehäuse statt. Die Beabstandung zwischen der Nadelplatte und dem umgebenden Gehäuse kann um die Nadelplatte herum umlaufend durchgehend ausgebildet sein.

Die Nadelplatte kann in der weiter zurückgestellten Nichtarbeitsstellung beabstandet von die Nadelplatte umgebenden Gehäuseabschnitten des Gehäuses freiliegend angeordnet sein. Die Nadelplatte ist hier berührungskontaktfrei in der weiter zurückgestellten Nichtarbeitsstellung angeordnet.

Das Microneedling-Handgerät kann mit einem wiederverwendbaren Antriebsmodul, in welchem die Antriebseinrichtung angeordnet ist, und einem Einwegmodul gebildet sein, welches mit dem Antriebsmodul lösbar verbunden ist und in dem die Hautstecheinrichtung angeordnet ist. Das Antriebsmodul und das Einwegmodul können mit einem jeweils zugeordneten Modulgehäuse gebildet sein, wobei die Modulgehäuse lösbar miteinander gekoppelt oder verbunden werden können.

Die Antriebseinrichtung kann eingerichtet sein, das Microneedling-Stechwerkzeug mittels der hierauf eingeleiteten Antriebskraft im Betrieb mit einer Wiederholfrequenz zwischen etwa 10 Hz und etwa 200 Hz vor und zurück zu bewegen, alternativ mit einer Wiederholfrequenz zwischen etwa 25 Hz und etwa 160 Hz.

Es kann vorgesehen sein, dass zwischen der vorderen Arbeitsstellung und der im Vergleich hierzu zurückgestellten hinteren Arbeitsstellung in Längsrichtung des Microneedling-Handgeräts ein Abstand (Arbeitshublänge) von etwa 0,5 mm bis etwa 5 mm gebildet ist. Alternativ kann vorgesehen sein, dass zwischen der vorderen Arbeitsstellung und der im Vergleich hierzu zurückgestellten hinteren Arbeitsstellung in Längsrichtung des Microneedling-Handgeräts ein Abstand (Arbeitshublänge) von lediglich etwa 1 mm bis etwa 4 mm gebildet ist. Mittels unterschiedlicher Hublängen ist das Microneedling-Handgerät für unterschiedliche Anwendungen konfigurierbar, zum Beispiel für medizinisches oder kosmetisches Microneedling.

Bei dem Microneedling-Handgerät kann eine Schutz- oder Abdeckkappe vorgesehen sein, die am Gehäuse lösbar und die Nadelplatte abdeckend angeordnet ist, wobei an der Schutzkappe außen ein Funktionswerkzeug angeordnet ist. Zum Beispiel können an der Schutzkappe außen, insbesondere im Bereich einer Fronfläche, mehrere Hautmassagevorsprünge angeordnet sein, die auf der Außenfläche der Schutzkappe verteilt angeordnet sind. Zum Beispiel können die Hautmassagevorsprünge ein abgerundetes Kopfteil aufweisen, beispielsweise mit einem Kugelkopf.

An der Abdeckkappe, welche im aufgesetzten Zustand die Stechnadeln abdeckt, ist so ein Funktionswerkzeug angeordnet, welches mit einer Anordnung von Funktionselementen gebildet sein kann, bei denen es sich beispielsweise um Massageelemente handelt. Die Abdeckkappe ist so als Multifunktionskappe ausführbar.

An der Abdeckkappe können Öffnungen vorgesehen sein. Zum Beispiel sind die Öffnungen im Bereich einer Mantelfläche der Abdeckkappe angeordnet und bilden so seitliche Öffnungen. Alternativ oder ergänzend können solche Öffnungen bei anderen Ausführungsformen im Bereich der Frontfläche angeordnet sein. Zum Beispiel können die Öffnungen dazu dienen, für die Nadelplatte mit den mehreren Stechnadeln bei aufgesetzter Abdeckkappe eine Gassterilisation durchzuführen. Seitliche Öffnungen haben den Vorteil, dass der Eintritt von Staubpartikeln zu der Nadelplatte und hierdurch verursachte Staubablagerungen gemindert werden.

Die Nadelplatte kann in einer Ausgestaltung mit einer Wendeplatte oder einem Wendeeinsatz ausgeführt sein, derart, dass zum Beispiel unterschiedliche Microneedling-Stechwerkzeuge hieran jeweils wechsel- oder lösbar angeordnet werden können, um unterschiedliche (Microneedling-)Funktionen bereitzustellen und / oder einen Verschleiß des Stechwerkzeugs zu kompensieren. Es kann sich auch um Wechseleinsätze handeln, die je nach gewünschter Anwendung an der Nadelplatte lösbar angeordnet werden. Wendeeinsätze können auf beiden Seiten Stechwerkzeuge tragen. Die Wechseleinsätze können aus einem Sortiment oder einem abgestuften Satz von Werkzeugeigenschaften auswählbar sein.

Die vorangehend in Verbindung mit dem Microneedling-Handgerät erläuterten Ausgestaltungen können im Zusammenhang mit der Hautstecheinrichtung und / oder dem Artikel entsprechend vorgesehen sein.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Microneedling-Handgeräts;
- Fig. 2: eine schematische perspektivische Darstellung des Microneedling-Handgeräts aus Fig. 1., wobei ein Stechmodul mit einem Microneedling-Stechwerkzeug gelöst ist;
- Fig. 3: eine schematische perspektivische Darstellung eines vorderen Abschnitts des Microneedling-Handgeräts aus Fig. 1, wobei das Microneedling-Stechwerkzeug mit Nadelplatte in einer vorderen Arbeitsstellung angeordnet ist;
- Fig. 4: eine schematische perspektivische Darstellung des vorderen Abschnitts des Microneedling-Handgeräts, wobei das Microneedling-Stechwerkzeug mit Nadelplatte in einer zurückgestellten hinteren Arbeitsstellung angeordnet ist;
- Fig. 5: eine schematische perspektivische Darstellung des vorderen Abschnitts des Microneedling-Handgeräts, wobei vorderseitig auf das Microneedling-Stechwerkzeug die Nadelplatte abdeckend eine Abdeckkappe aufgesetzt ist;
- Fig. 6: eine schematische perspektivische Darstellung des vorderen Abschnitts des Microneedling-Handgeräts teilweise im Schnitt mit aufgesetzter Abdeckkappe;
- Fig. 7: eine schematische perspektivische Darstellung des vorderen Abschnitts des Microneedling-Handgeräts teilweise im Schnitt mit abgenommener Abdeckkappe;
- Fig. 8: eine schematische perspektivische Darstellung einer elektrischen Zahnbürste;
- Fig. 9: eine schematische perspektivische Darstellung eines weiteren Microneedling-Handgeräts;
- Fig. 10: eine schematische perspektivische Darstellung eines vorderen Gehäuseteils des Microneedling-Handgeräts aus Fig. 9 im Schnitt;
- Fig. 11: eine schematische perspektivische Darstellung des vorderen Gehäuseteils des Microneedling-Handgeräts aus Fig. 9, wobei die Nadelplatte des Stechwerkzeugs in einer eingefahrenen Nichtarbeitsstellung angeordnet ist;
- Fig. 12: eine schematische perspektivische Darstellung des vorderen Gehäuseteils des Microneedling-Handgeräts aus Fig. 9, wobei die Nadelplatte des Stechwerkzeugs in einer vorderen Arbeitsstellung freiliegend angeordnet ist;
- Fig. 13: eine schematische perspektivische Darstellung des vorderen Gehäuseteils des Microneedling-Handgeräts aus Fig. 9, wobei die Nadelplatte in einer zurückgestellten hinteren Arbeitsstellung freiliegend angeordnet ist; und
- Fig. 14: eine schematische perspektivische Darstellung der Abdeck- oder Schutzkappe

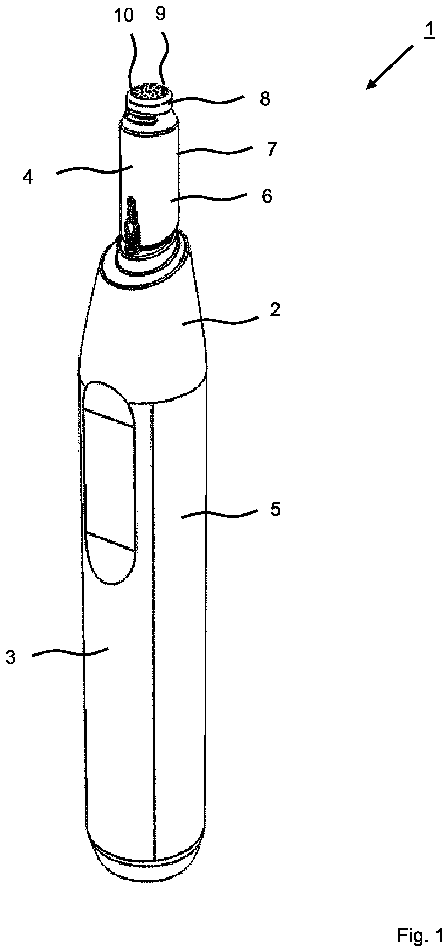

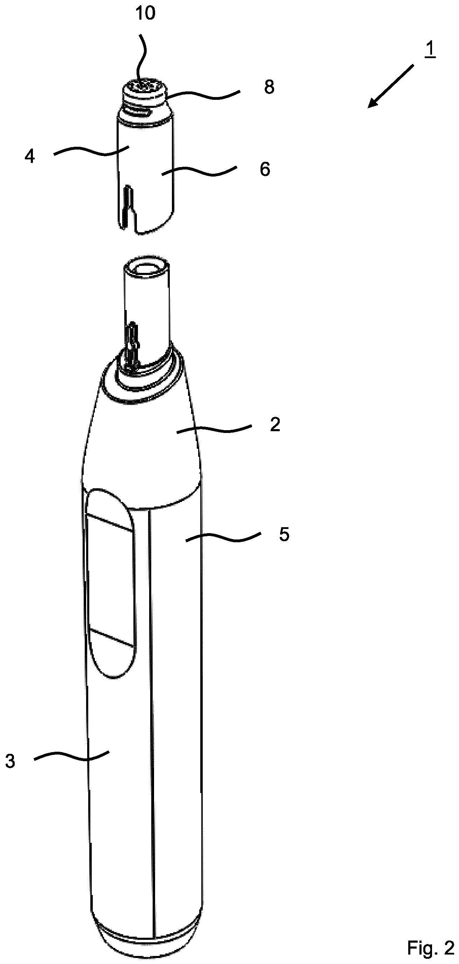

Fig. 1 bis 7 zeigen perspektivische Darstellungen für ein Microneedling-Handgerät 1, welches ein Gehäuse 2 aufweist, welches im gezeigten Beispiel mehrstückig ausgeführt ist. Das Gehäuse 2 ist mit einem hinteren Gehäuseteil 3 und einem vorderen Gehäuseteil 4 gebildet, die einem Antriebsmodul 5 und einer hieran lösbar angeordneten Hautstecheinrichtung 6 zugeordnet sind, mit dem wahlweise ein Stechmodul 7 gebildet ist. Gemäß Fig. 2 ist die Hautstecheinrichtung 6 und somit wahlweise das Stechmodul 7 von dem Antriebsmodul 5 abnehmbar und auf diese Weise wechselbar. Bei dem gezeigten Beispiel sind Antriebsmodul 5 und Hautstecheinrichtung 6 mit einer Steckverbindung lösbar verbunden.

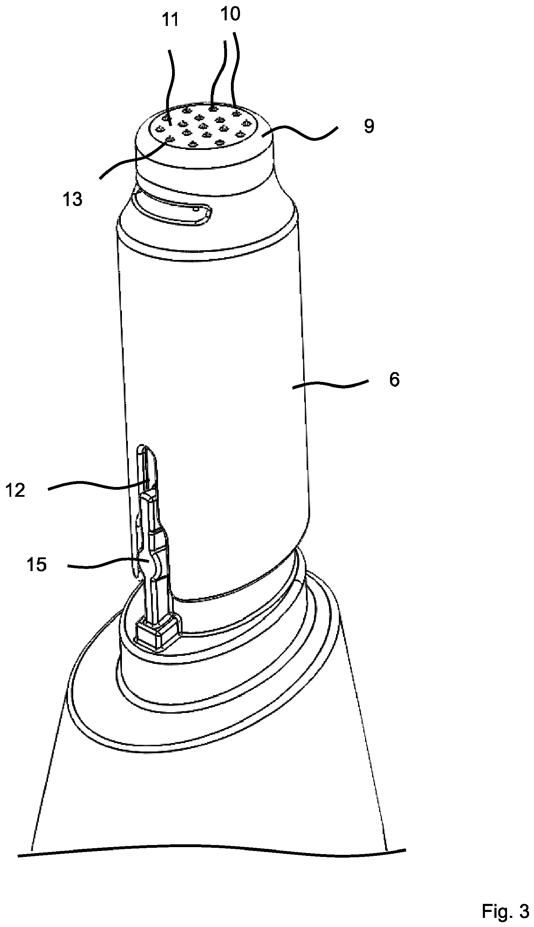

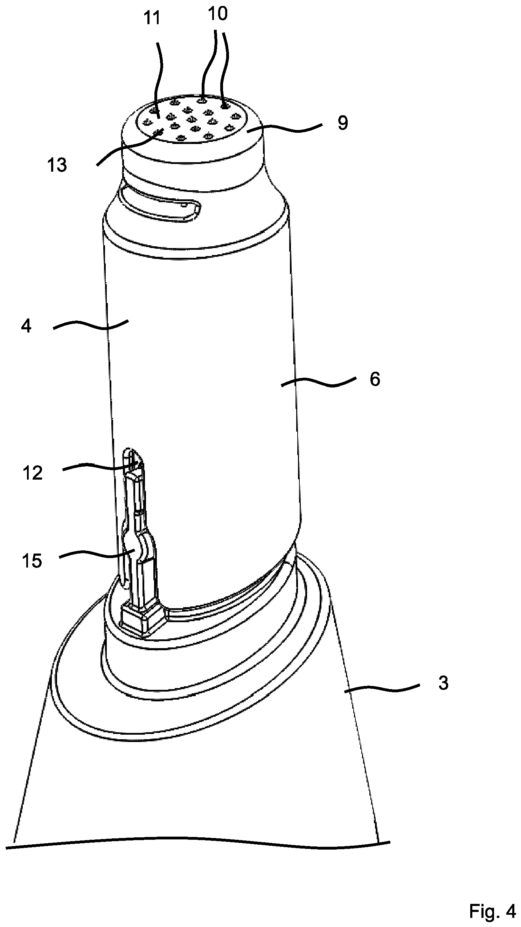

In dem hinteren Gehäuseteil 3, welches dem Antriebsmodul 5 zugeordnet ist, ist eine Antriebseinrichtung angeordnet, beispielsweise ein Elektromotor, mit der eine Antriebsbewegung oder -kraft bereitgestellt wird, die auf ein Microneedling-Stechwerkzeug 8 der Hautstecheinrichtung 6 gekoppelt wird, um in Betrieb das Microneedling-Stechwerkzeug 8 mittels der bereitgestellten Antriebskraft mit einer Wiederholfrequenz in Längsrichtung des Gehäuses 2 vor- und zurück zu bewegen. Auf diese Weise wird das Microneedling-Stechwerkzeug 8, welches mit einer Nadelplatte 9 gebildet ist, gemäß den Fig. 3 und 4 zwischen einer vorderen Arbeitsstellung (Fig. 3) und einer hinteren Arbeitsstellung (Fig. 4) repetierend verlagert. Auf diese Weise werden im Betrieb mehrere Stechnadeln 10, die an der Nadelplatte 9 im Bereich einer Anwendungsfläche 11 vorstehen lokal in einen zugeordneten Hautabschnitt eingestochen und wieder zurückgezogen. Die Nadelplatte 9 mit den mehreren Stechnadeln 10 ist hierbei sowohl in der vorderen als auch in der hinteren Arbeitsstellung gemäß den Fig. 3 und 4 freiliegend, also insbesondere nicht von einem Abschnitt des Gehäuses 2 ganz oder teilweise umgeben und somit außerhalb eines Innenraums 12 des Gehäuses 2 angeordnet. Auf diese Weise kann der lokale Hautabschnitt, welcher mit den mehreren Stechnadeln 10 in Kontakt tritt, aufgrund des Vor- und Zurückbewegens des Microneedling-Stechwerkzeugs 8 mit der Nadelplatte 9 frei schwingen, was ein effizientes und möglichst schmerzfreies Aufstechen der Haut unterstützt.

Im gezeigten Beispiel sind die mehreren Stechnadeln 10 in zugeordneten Öffnungen 13 im Bereich der Anwendungsfläche 11 an der Nadelplatte 9 angeordnet und hierin fixiert, beispielsweise mittels Kleben oder Löten. In einer anderen Ausführungsform können die mehreren Stechnadeln 10 auf die Anwendungsfläche 11 aufgesetzt werden, beispielsweise mittels Aufschweißen. Alternativ können die Stechnadeln 10 mittels Spritzgussverfahren angeformt sein oder als Einlegebauteile integriert werden. Die mehreren Stechnadeln können oberhalb der Anwendungsfläche 11 alle eine im Wesentlichen gleiche Nadellänge oder unterschiedliche Nadellängen aufweisen, wobei hierbei Gruppen der mehreren Stechnadeln 10 von gleicher Nadellänge sein können.

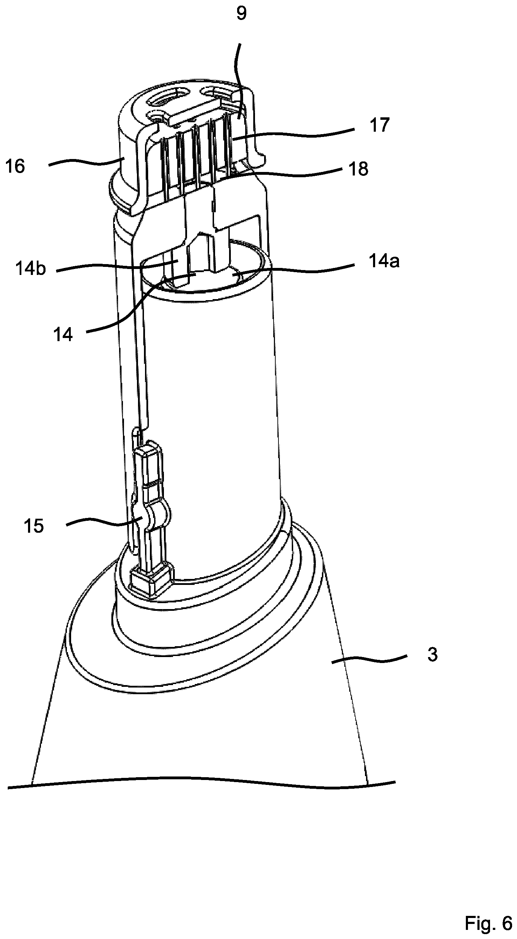

Bei dem gezeigten Beispiel in den Fig. 1 bis 7 ist die Hautstecheinrichtung 6, somit wahlweise das Stechmodul 7, mittels Kopplung 14 aufgesteckt, die mit einem antriebsseitigen Kopplungsteil 14a und einem stechwerkzeugseitigen Kopplungsteil 14b gebildet ist. Die Kopplung 14 ist beispielweise als eine Magnetkopplung zwischen dem antriebsseitigen Kopplungsteil 14a und dem stechwerkzeugseitigen Kopplungsteil 14b ausgebildet (vgl. Fig. 6). Über die Kopplung 14 erfolgt die Einleitung der Antriebskraft / -bewegung zum Vor- und Zurückbewegen der Nadelplatte 9 mit den Stechnadeln 10.

Bei dem gezeigten Beispiel ist eine Verdrehsicherung 15 vorgesehen, welche das Stech- oder Stechwerkzeugmodul 6 gegen Verdrehen sichert.

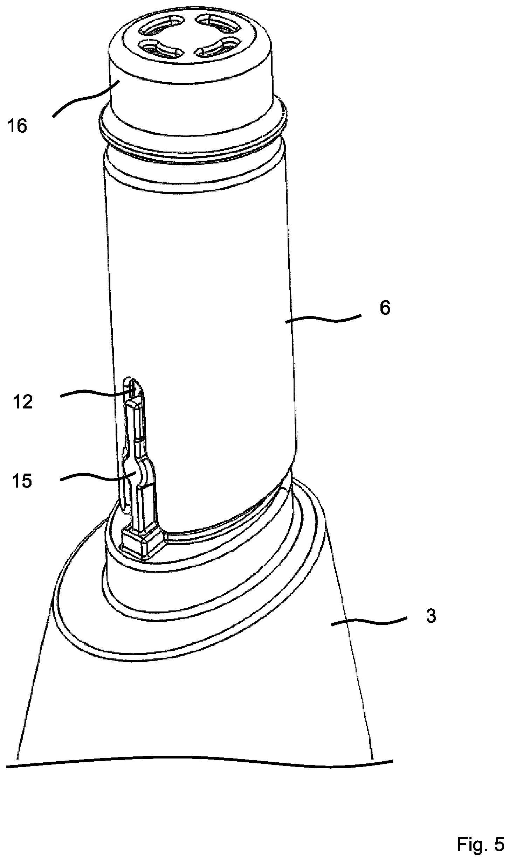

Gemäß Fig. 5 kann die Nadelplatte 9 mit den mehreren Stechnadeln 10 im Nichtbetrieb mittels einer Abdeckkappe 16 abgedeckt werden, die lösbar aufgesetzt und so zum Betrieb abnehmbar ist. Beim gezeigten Beispiel ist die Abdeckkappe 16 aufgesteckt.

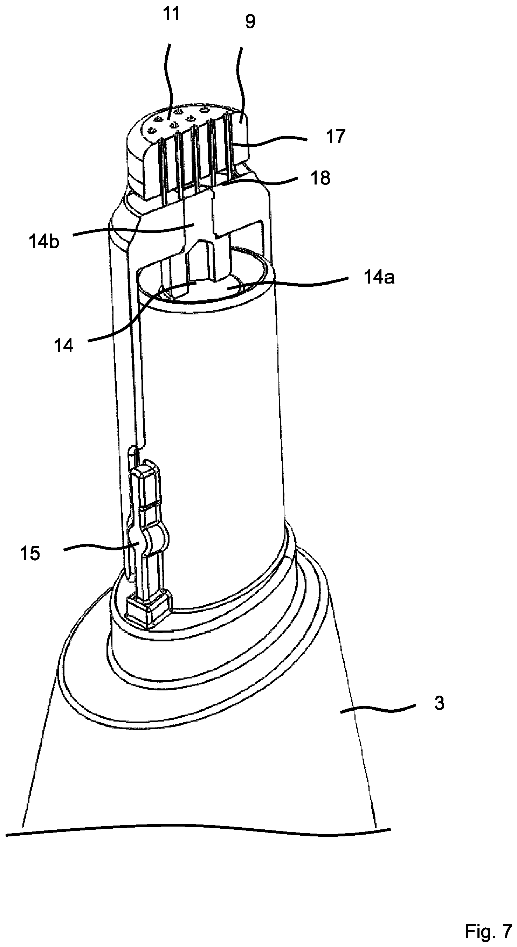

Fig. 6 und 7 zeigen den vorderen Abschnitt des Microneedling-Handgeräts 1 teilweise im Schnitt mit und ohne die Abdeckkappe 16. Es ergibt sich, dass die mehreren Stechnadeln 10 in zugeordneten Bohrungen 17 der Nadelplatte 9 verlaufen und rückseitig zur Nadelplatte 9 an einem Nadelhalter 18 aufgenommen sind.

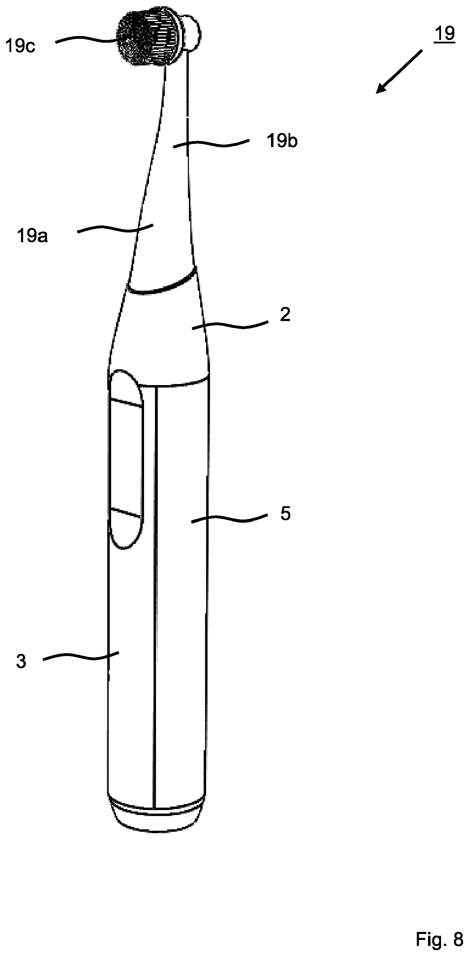

Fig. 8 zeigt eine schematische perspektivische Darstellung einer elektrischen Zahnbürste 19, bei ein vorderer Gehäuseteil 19a mit einem Zahnbürstenaufsatz 19b gebildet ist, welcher anstelle der Hautstecheinrichtung 6 aufgesetzt ist und an die Antriebseinrichtung im hinteren Gehäuseteil 3 des Gehäuses 2 koppelt, so dass die Antriebskraft (Vor- und Zurückbewegung) genutzt wird, um eine Bürstenanordnung 19c im Betrieb zu bewegen. Hautstecheinrichtung 6 und Zahnbürstenaufsatz 19b können so wahlweise lösbar aufgesetzt und mit ein und demselben Antriebsmodul 5 betrieben werden, so dass entweder das Microneedling-Handgerät 1 oder die elektrische Zahnbürste 19 bereitgestellt ist.

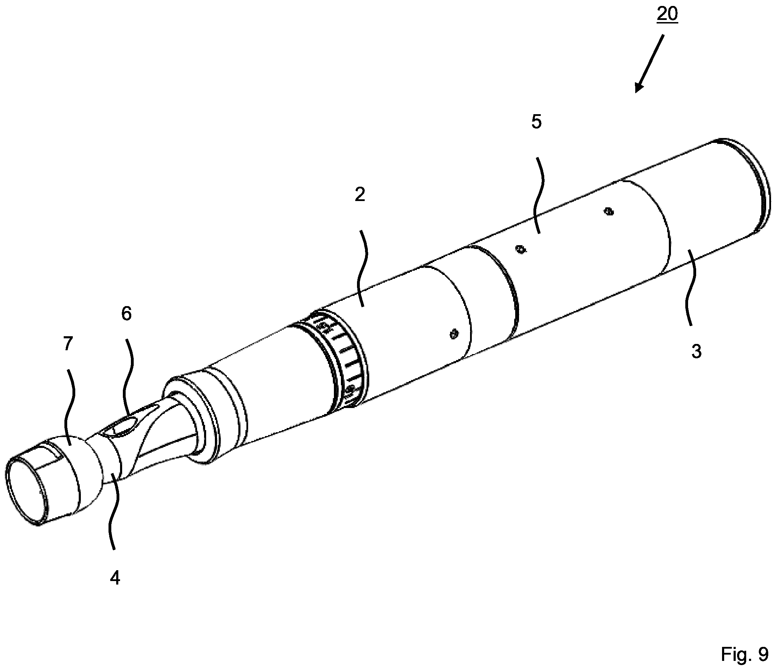

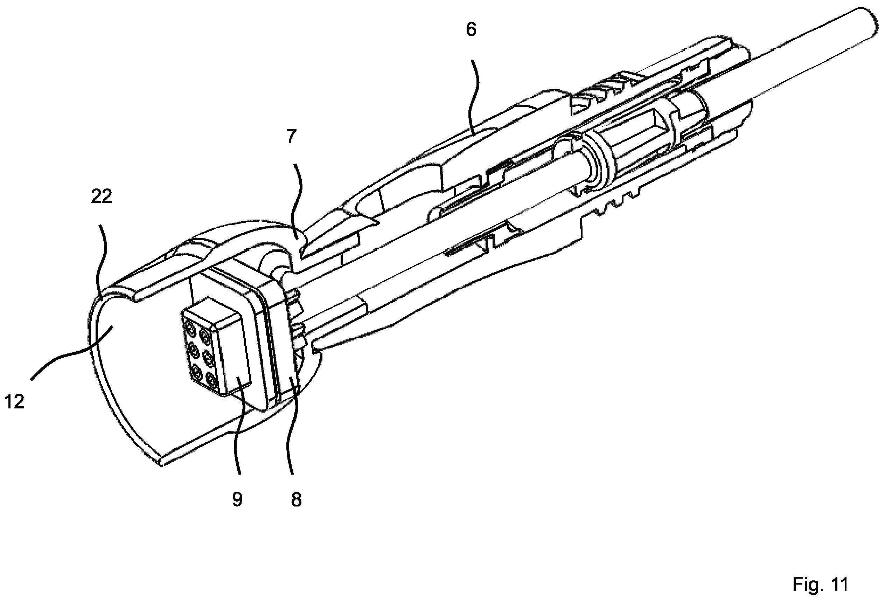

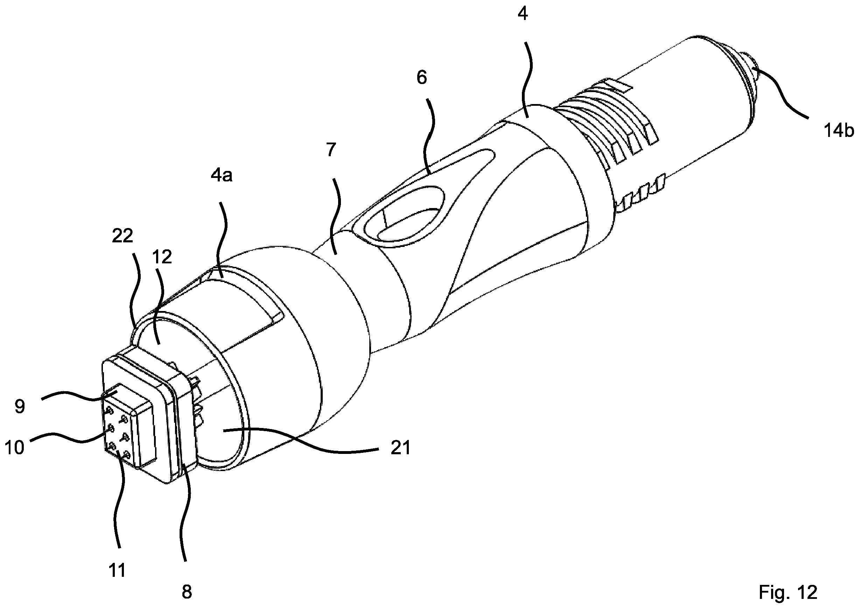

Fig. 9 bis 13 zeigen ein weiteres Microneedling-Handgerät 20, bei dem das Microneedling-Stechwerkzeug 8 mit der Nadelplatte 9 durch eine vorderseitige Gehäuseöffnung 21 an der Hautstecheinrichtung 6 (Stechmodul 7) zwischen einer eingefahrenen Nichtarbeitsstellung gemäß Fig. 9 bis 11 sowie ausgefahrenen Arbeitsstellungen gemäß den Fig. 11 und 12 verlagerbar ist.

Im Nichtbetrieb kann die Nadelplatte 9 der Hautstecheinrichtung 6 mit den hierauf angeordneten mehreren Stechnadeln 10 so hinter die Fläche der Gehäuseöffnung 21 zurückgefahren werden, derart, dass gemäß Fig. 9 sowohl Nadelplatte 9 wie auch Stechnadeln 10 vollständig hinter der Gehäuseöffnung 21 im Innenraum 12 des vorderen Gehäuseteils 4 angeordnet sind, welches bei der gezeigten Ausführungsform der Hautstecheinrichtung 6 zugeordnet ist. Am vorderen Gehäuseteils 4 ist die Gehäuseöffnung 21 an einem vorderen Abschnitt 4a gebildet.

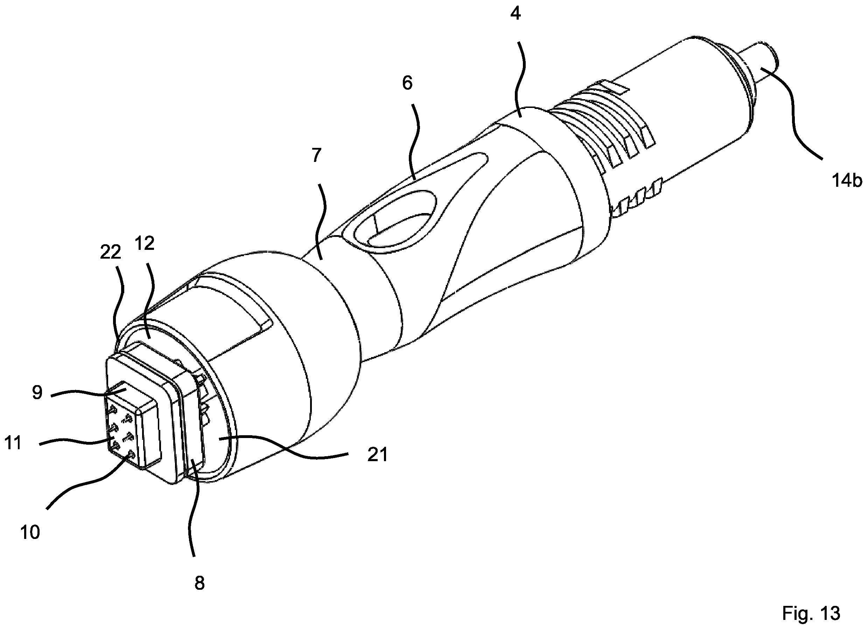

Im Betrieb fürs Microneedling wird die Nadelplatte 9 mit den mehreren Stechnadeln 10 gemäß den Fig. 12 und 13 zwischen der vorderen Arbeitsstellung (vgl. Fig. 12) und der hiergegenüber zurückgestellten hinteren Arbeitsstellung (vgl. Fig. 13) hin und her bewegt, also in Längsrichtung des Gehäuses 2 einem Arbeitshub entsprechend vor und zurück, so dass die Nadelplatte 9 mit den mehreren Stechnadeln 10 sowohl in der vorderen wie auch in der hinteren Arbeitsstellung in Bezug auf die Gehäuseöffnung 21 vor deren Fläche und somit außerhalb des Gehäuses 2 angeordnet ist, insbesondere außerhalb des vorderen Abschnitts 4a des vorderen Gehäuseteils 4, was wiederum ein Anregen des zu bearbeitenden Hautabschnitts zum freien Schwingen ermöglicht, also frei von einer Behinderung dieser Schwingung durch das Gehäuse 2, insbesondere einen die Gehäuseöffnung 21 umgebenden vorderen Gehäuserand 22. Bei dem gezeigten Beispiel ist im Betrieb im Wesentlichen das gesamte Microneedling-Stechwerkzeug 8 vor der Gehäuseöffnung 21 angeordnet. Im Nichtbetrieb kann die Nadelplatte 9 dann in die eingefahrene Nichtarbeitsstellung gemäß Fig. 9 bis 10 verlagert werden.

Gemäß dem Beispiel in den Fig. 9 bis 13 ist die Nadelplatte 9 beim Hindurchführen durch die Gehäuseöffnung 21 umlaufend und durchgehend beabstandet vom Gehäuserand 22, also kontaktberührungsfrei.

Es kann vorgesehen sein, dass die Nadelplatte 9 innerhalb des vorderen Gehausteils 4 hinter der Gehäuseöffnung 21 mehrere unterschiedliche eingefahrene Nichtarbeitsstellungen einnehmen kann, die sich hinsichtlich Ihres jeweiligen Abstands zur Gehäuseöffnung unterscheiden.

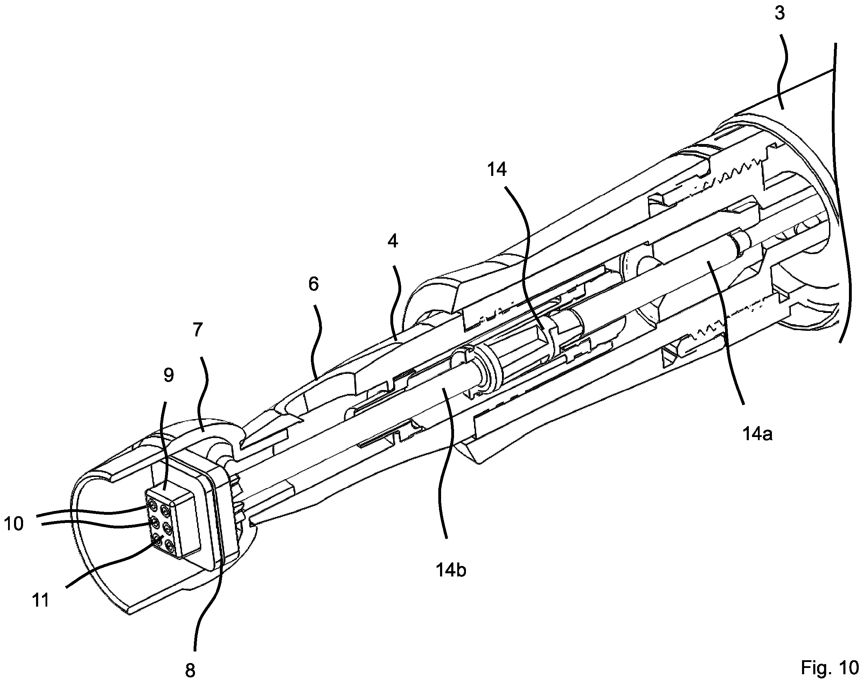

Im Bereich der Kopplung 14 ist das rückseitige, stechwerkzeugseitige Kopplungsteil 14b der Hautstecheinrichtung 6 gemäß Fig. 10 mit dem hier als Schaftbauteil ausgebildeten antriebsseitigen Kopplungsteil 14a (lösbar) verbunden, um die bereitgestellte Antriebskraft / -bewegung auf das Microneedling-Stechwerkzeug 8 mit der Nadelplatte 9 einzukoppeln.

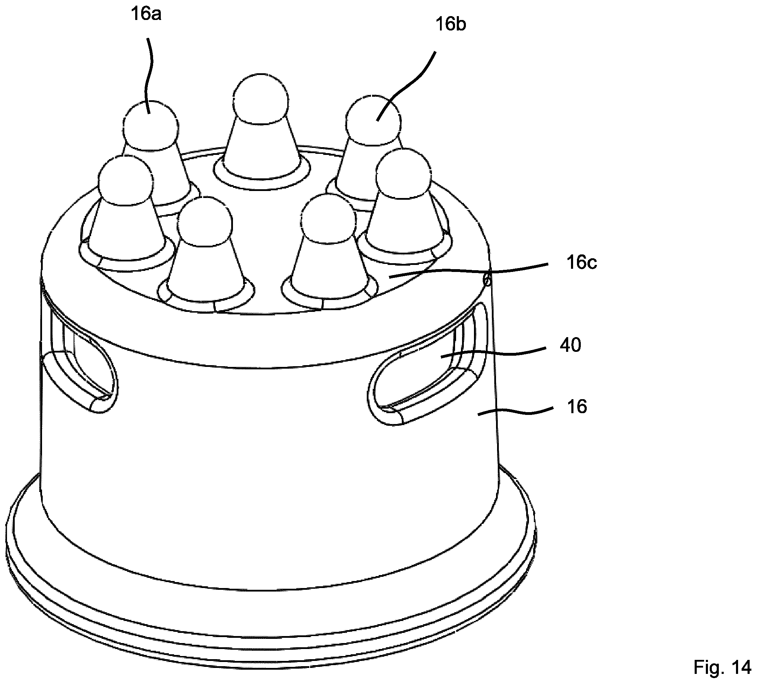

Fig. 14 zeigt eine vergrößerte perspektivische Darstellung einer anderen Ausführung der Schutz- oder Abdeckkappe 16 mit einem Funktionswerkzeug 16a. An der Abdeckkappe 16 sind Öffnungen 40 vorgesehen. Im gezeigten Beispiel sind die Öffnungen im Bereich einer Mantelfläche 41 der Abdeckkappe 16 angeordnet und bilden so seitliche Öffnungen. Alternativ oder ergänzend können solche Öffnungen bei anderen Ausführungsformen im Bereich der Frontfläche 16c angeordnet sein (vgl. Fig. 5). Zum Beispiel können die Öffnungen 40 dazu dienen, für die Nadelplatte 9 mit den mehreren Stechnadeln 10 bei aufgesetzter Abdeckkappe 16 eine Gassterilisation durchzuführen. Die in Fig. 14 gezeigten seitlichen Öffnungen 40 haben den Vorteil, dass der Eintritt von Staubpartikeln zu der Nadelplatte 9 und hierdurch verursachte Staubablagerungen gemindert sind.

An der Abdeckkappe 16, welche im aufgesetzten Zustand die mehreren Stechnadeln 10 abdeckt, ist das Funktionswerkzeug 16a angeordnet, welches bei dem dargestellten Ausführungsbeispiel mit einer Anordnung von Funktionselementen 16b gebildet ist, bei denen es sich beispielweise um Massageelemente handelt. Die Abdeckkappe 16 ist so als Multifunktionskappe ausgeführt. Beim dargestellten Ausführungsbeispiel sind die Funktionselemente 16b insbesondere im Bereich einer Front- oder Stirnfläche 16c der Abdeckkappe 16 ausgebildet.

Die Abdeckkappe 16 kann alternativ, zumindest außen, eine geneigte Stirnfläche mit Funktionswerkzeug aufweisen. Auch wegklappbar ist denkbar, oder auch eine axial verschiebliche Hülse mit fehlender oder für das Funktionswerkzeug durchbrochener Deckfläche (vergleichbar einer Art Bohrkrone mit Werkzeugen am Umfang oder auf der Restdeckfläche), zu- / abschaltbar bspw. durch eine Drehkulisse-Kurvensteuerung per Dreh-Schiebe-Bewegung.

Andere Funktionswerkzeuge können an der Abdeckkappe 16 vorgesehen sein, beispielsweise Silikonnoppen, Greif-Kneif-Werkzeuge aus elastischem Material und / oder abwechselnd gerichtete Stimulationswerkzeuge, die ihrerseits eine stech-ähnliche (Stechen simulierend) Wirkung auf der Haut erzielen und hierbei eine Hautspreizung bewirken. Alternative Funktionswerkzeuge zur Hautstimulierung können vorgesehen sein, die sich beispielweise bezüglich eines Spitzenradius oder -winkels von den vorgenannten unterscheiden.

Die Nadelplatte 9 kann in einer Ausgestaltung als Wendeplatte oder Wendeeinsatz ausgeführt sein, derart, dass unterschiedliche Funktionswerkzeuge hieran jeweils wechsel- oder lösbar angeordnet werden, um mehrere Funktionen bereitzustellen oder einen Verschleiß zu kompensieren. Es kann sich auch um Wechseleinsätze handeln, die je nach gewünschter Anwendung an der Nadelplatte 9 lösbar angeordnet werden. Wendeeinsätze können auf beiden Seiten Werkzeuge tragen. Die Wechseleinsätze können aus einem Sortiment oder einem abgestuften Satz von Werkzeugeigenschaften auswählbar sein.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Microneedling-Handgerät (1; 20) zum lokalen Aufstechen einer Haut, mit
- einem Gehäuse (2);
- einer Antriebseinrichtung, die in dem Gehäuse (2) angeordnet und eingerichtet ist, eine Antriebskraft bereitzustellen;
- einer Hautstecheinrichtung (6);
- einem Microneedling-Stechwerkzeug (8), das an der Hautstecheinrichtung (6) gebildet und mit der Antriebseinrichtung verbunden ist, derart, dass das Microneedling-Stechwerkzeug (8) mittels der Antriebskraft im Betrieb mit einer Wiederholfrequenz vor und zurück bewegbar ist; und
- einer Nadelplatte (9), die an dem Microneedling-Stechwerkzeug (8) gebildet ist und bei der über eine vorderseitige Anwendungsfläche (11) verteilt mehrere Stechnadeln (10) angeordnet sind;
wobei das Microneedling-Stechwerkzeug (8) mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar ist, wobei die Hautstecheinrichtung (6) an dem Gehäuse (2) lösbar angeordnet ist und wobei die Antriebseinrichtung mit einer Antriebseinrichtung für eine elektrische Zahnbürste gebildet ist, welche eingerichtet ist, hieran wahlweise die Hautstecheinrichtung (6) und einen mittels der Antriebseinrichtung betreibbaren Zahnbürstenaufsatz lösbar zu koppeln.

2. Microneedling-Handgerät (1; 20) nach Anspruch 1, **dadurch gekennzeichnet, dass** beim Bewegen zwischen der vorderen Arbeitsstellung und der hinteren Arbeitsstellung zumindest die mehreren Stechnadeln (10) der Nadelplatte (9) in der vorderen Arbeitsstellung und der hinteren Arbeitsstellung des Microneedling-Stechwerkzeugs (8) vollständig außerhalb des Gehäuses (2) und so freiliegend angeordnet sind.

3. Microneedling-Handgerät (1; 20) nach Anspruch 1 oder 2, dadurch **gekennzeich**- **net**, dass im Betrieb weiterhin zumindest die vorderseitige Anwendungsfläche (11) in der vorderen Arbeitsstellung und der zurückgestellten hinteren Arbeitsstellung des Microneedling-Stechwerkzeugs (8) vollständig außerhalb des Gehäuses (2) und so freiliegend angeordnet ist.

4. Microneedling-Handgerät (1; 20) nach mindestens einem der vorangehenden Ansprüche, **dadurch gekennzeichnet, dass** bei dem Microneedling-Stechwerkzeug (8) die Nadelplatte (9) frei von einem die Nadelplatte (9) zumindest teilweise umgreifenden Gehäuseabschnitt des Gehäuses (2) gebildet ist.

5. Microneedling-Handgerät (1; 20) nach Anspruch 4, **dadurch gekennzeichnet, dass** die Nadelplatte (9) an einem Gehäuseabschnitt des Gehäuses (2) angeordnet ist, welcher im Betrieb beim Vor- und Zurückbewegen des Microneedling-Stechwerkzeugs (8) mit diesem zusammen mitbewegt wird.

6. Microneedling-Handgerät (1; 20) nach Anspruch 4, **dadurch gekennzeichnet, dass** die Nadelplatte (9) an dem Gehäuseabschnitt des Gehäuses (2) einstückig angeformt ist.

7. Microneedling-Handgerät (1; 20) nach mindestens einem der vorangehenden Ansprüche, **dadurch gekennzeichnet, dass** ein vorderes Gehäuseteil (4), welches der Hautstecheinrichtung (6) zugeordnet ist, rückwärtig hülsenförmig gestaltet ist und einen hinteren Gehäuseteil (3) zumindest teilweise umschließt, welcher der Antriebseinrichtung zugeordnet ist.

8. Microneedling-Handgerät (1; 20) nach mindestens einem der vorangehenden Anspruch, soweit auf Anspruch 4 oder 5 rückbezogen, **dadurch gekennzeichnet, dass** das Microneedling-Stechwerkzeug (8) zusammen mit dem Gehäuseabschnitt von dem Gehäuse (2) lösbar ist.

9. Microneedling-Handgerät (1; 20) nach mindestens einem der vorangehenden Ansprüche, **dadurch gekennzeichnet, dass** das Microneedling-Stechwerkzeug (8) durch eine Öffnung (21) an einem vorderseitigen Gehäuseende des Gehäuses (2) hindurch in eine im Vergleich zur zurückgestellten hinteren Arbeitsstellung weiter zurückgestellte Nichtarbeitsstellung verlagerbar ist, in welcher zumindest die vorderseitige Anwendungsfläche der Nadelplatte (9) gegenüber der Öffnung (21) zurücksteht.

10. Microneedling-Handgerät (1; 20) nach Anspruch 9, **dadurch gekennzeichnet, dass** die Nadelplatte (9) beim Verlagern durch die Öffnung (21) hindurch ein- oder mehrseitig von einem die Öffnung (21) umgebenden Rand (22) beabstandet ist.

11. Microneedling-Handgerät (1; 20) nach Anspruch 9 oder 10, **dadurch gekennzeichnet, dass** die Nadelplatte (9) in der weiter zurückgestellten Nichtarbeitsstellung beabstandet von die Nadelplatte (9) umgebenden Gehäuseabschnitten des Gehäuses (2) freiliegend angeordnet ist.

12. Microneedling-Handgerät (1; 20) nach mindestens einem der vorangehenden Ansprüche, **gekennzeichnet durch** ein wiederverwendbares Antriebsmodul (4), in welchem die Antriebseinrichtung angeordnet ist, und ein Einwegmodul (5), welches mit dem Antriebsmodul lösbar verbunden ist und in dem die Hautstecheinrichtung (8) angeordnet ist.

13. Microneedling-Handgerät (1; 20) nach mindestens einem der vorangehenden Ansprüche, **dadurch gekennzeichnet, dass** die Antriebseinrichtung eingerichtet ist, das Microneedling-Stechwerkzeug (8) mittels der hierauf eingeleiteten Antriebskraft im Betrieb mit einer Wiederholfrequenz zwischen etwa 10 Hz und etwa 200 Hz vor und zurück zu bewegen.

14. Artikel, mit
- einer Antriebseinrichtung, die in einem Gehäuse angeordnet ist, welches wahlweise ein Handstück ausbildend ausgeführt ist;
- einem Zahnbürstenaufsatz, welcher zum Ausbilden einer elektrischen Zahnbürste mit dem Gehäuse lösbar und so an die Antriebseinrichtung koppelnd verbunden werden kann; und
- einer Hautstecheinrichtung (6), welche zum Ausbilden eines Microneedling-Handgeräts (1; 20) mit dem Gehäuse lösbar und so an die Antriebseinrichtung koppelnd verbunden werden kann.
